# EUROPÄISCHE PATENTSCHRIFT

(11) **EP 4 055 135 B1**
(45) Veröffentlichungstag und Bekanntmachung des Hinweises auf die Patenterteilung: **08.07.2026**
(21) Anmeldenummer: 20828259.0
(22) Anmeldetag: 04.11.2020
(51) Int. Cl.: C12M 1/00, C12M 1/12, C12M 1/26, C12M 1/34

(54) **VERFAHREN UND EINRICHTUNG ZUR PRODUKTION VON MIKROALGENBIOMASSE**
METHOD AND DEVICE FOR THE PRODUCTION OF MICROALGAE BIOMASS
PROCÉDÉ ET DISPOSITIF DE POUR PRODUIRE DE LA BIOMASSE DE MICROALGUES

(30) Priorität: 07.11.2019 DE 102019130109
(43) Veröffentlichungstag der Anmeldung: 14.09.2022
(73) Patentinhaber: IGV Institut für Getreideverarbeitung GmbH, 14558 Nuthetal (DE)
(72) Erfinder: HUSCHEK, Gerd, 16552 Schildow (DE); THEISEN, Horst, 53117 Bonn (DE)
(74) Vertreter: Maucher Jenkins Patent- und Rechtsanwälte
(86) Internationale Anmeldenummer: PCT/DE2020/100939
(87) Internationale Veröffentlichungsnummer: WO 2021/089085

(56) Entgegenhaltungen:
- EP-B1- 2 446 016
- DE-A1- 102014 012 217
- DE-A1- 102017 109 968
- US-A1- 2011 312 062
- US-B2- 10 023 830

## Beschreibung

Die Erfindung betrifft eine Lösung zur Produktion von Mikroalgenbiomasse, welche in besonders vorteilhafter Weise eine Großproduktion, nämlich die Produktion sehr großer Mengen von Mikroalgenbiomasse im industriellen Maßstab ermöglicht. Die vorgestellte Lösung ermöglicht insbesondere eine jahreszeitenunabhängige Großproduktion von Mikroalgenbiomasse bei einem gleichzeitig verhältnismäßig geringen Flächenbedarf. Gegenstände der Erfindung sind ein entsprechendes Verfahren und eine zu dessen Durchführung geeignete Einrichtung zur Produktion von Mikroalgenbiomasse.

Seit einigen Jahren ist eine auch gegenwärtig noch steigende Nachfrage nach Mikroalgenbiomasse zu verzeichnen. Mikroalgen, das heißt deren gegebenenfalls mit ausgewählten sekundären Inhaltsstoffen, wie beispielsweise Vitaminen oder Omega-3-Fettsäuren oder Mineralien oder bioaktiven Proteinen/Peptiden, angereicherte Biomasse wird unter anderem in Nahrungsmitteln, in Mitteln zur Nahrungsergänzung, zu medizinischen Zwecken und in der Kosmetik in unterschiedlichster Form verwendet.

Um den insoweit bestehenden Bedarf an Mikroalgenbiomasse decken zu können, sind in der jüngeren Vergangenheit immer größere Einrichtungen zur Erzeugung großer Mengen von Mikroalgenbiomasse errichtet worden. Dabei werden die die Biomasse liefernden Mikroalgen in einer aus Wasser und den jeweiligen Mikroalgen bestehenden Suspension phototroph oder mixotroph kultiviert. Die Kultivierung der Mikroalgen erfolgt unter Zugabe von Nährstoffen und gegebenenfalls weiteren, in den Mikroalgen anzureichernden Stoffen in die Suspension, indem die Suspension über längere Zeiten hinweg für das Wachstum der Mikroalgen zwingend erforderlichem Licht ausgesetzt und ihr Nährstoffe, wie insbesondere CO₂ als gasförmiger Nährstoff, zugeführt werden. Um eine längere Exposition der Suspension mit Licht und Nährstoffen (insbesondere CO₂) zu ermöglichen, wird die Suspension bei dafür gebräuchlichen Verfahren üblicherweise in einem Photobioreaktor entlang längerer Wege oder in einem mehrfach durchlaufenen Kreislauf geführt.

Wenn sich die Mikroalgen aufgrund anhaltender Exposition mit Licht und Nährstoffen im ausreichenden Maße vermehrt haben, wird die Suspension oder werden jedenfalls Teile davon zur Trennung der in der Suspension enthaltenen Mikroalgen von den flüssigen Bestandteilen in der Regel in einer Zentrifuge ausgeschleudert. Die ausgeschleuderten Mikroalgen werden dann im Allgemeinen einem Trocknungsprozess unterzogen und schließlich entsprechend ihrem jeweils vorgesehenen Einsatzzweck weiterverarbeitet.

Zur Kultivierung von Mikroalgen, also ihrer über einen Zeitraum andauernden Exposition mit Licht und Nährstoffen, sind unterschiedliche Konzepte entwickelt worden. Beispielsweise werden hierfür sogenannte Rohr-Bioreaktoren verwendet, bei welchen eine die jeweiligen Mikroalgen enthaltende Suspension in einer Anordnung aus transparenten Rohren, nämlich in der Regel aus Glasrohren, entlang längerer Wege geführt und währenddessen, bei gleichzeitiger Einbringung von Nährstoffen in die Rohre, dem Licht ausgesetzt wird. Zur Exposition der Mikroalgen beziehungsweise der sie enthaltenen Suspension mit Licht wird häufig das Tageslicht verwendet, wobei gegebenenfalls auch eine Unterstützung durch künstliche Lichtquellen erfolgt. Ein solcher Rohr-Bioreaktor ist beispielsweise aus der DE 10 2009 028 474 A1 bekannt. Auch aus der US 10,023,830 B2 ist eine Lösung bekannt, bei welcher die Kultivierung der Produktion von Biomasse dienender Mikroalgen in einer, dazu vorzugsweise in einem Rohrreaktor oder auch in raceway ponds unter Einwirkung von Licht sowie dem Zusatz von Nährstoffen in einem Umlauf gehaltenen Suspension erfolgt. Dabei werden in der Suspension sensorisch unterschiedliche physikalische Größen fortwährend erfasst und basierend hierauf Prozessparameter im Sinne eines hohen Ertrags an Biomasse beeinflusst.

Ein anderes Konzept besteht darin, eine die Mikroalgen enthaltende Suspension in einer von Licht durchfluteten und mit CO₂ als gasförmigem Nährstoff versorgten Kammer mittels dazu geeigneter Düsen fein zu versprühen. Die Tröpfchen der im oberen Bereich einer solchen Kammer fein versprühten Suspension schweben in der Kammer allmählich zu Boden, wobei sie, beziehungsweise die in ihnen enthaltenen Mikroalgen, für die Dauer dieser Abwärtsbewegung dem Licht in der Kammer ausgesetzt sind. Am Boden der Kammer wird die Suspension in Form der dort zusammenlaufenden Tröpfchen gesammelt und dann mittels eines Rohrleitungs- und Pumpsystems wieder ihrer der Versprühung in der vorgenannten Kammer dienenden Düsen zugeführt. Nach einiger Zeit und einem vielfachen Durchlaufen des zuvor erläuterten Kreislaufs, verbunden mit einem entsprechenden Wachstum und einer Vermehrung der in der umlaufenden Suspension enthaltenen Mikroalgen, erfolgt wiederum die Ernte der Mikroalgen durch Ausschleudern aus der Suspension.

Ein das zuletzt erläuterte Prinzip nutzendes Verfahren sowie ein danach arbeitender Photobioreaktor werden zum Beispiel in der EP 2 446 016 B1 beschrieben. Auch die US 2011/0312062 A1 offenbart einen Photobioreaktor, der gemäß einer möglichen Ausbildungsform dieses Prinzip nutzt.

Aus der sich allgemein mit der phototrophen oder mixotrophen Kultivierung von Organismen oder Zellen befassenden Druckschrift EP 2 446 016 B1 ist es zudem bekannt, in der Kammer, in deren oberen Bereich die Suspension durch Versprühen ausgebracht wird, spezielle Strukturen anzuordnen, durch welche die gravitationsbedingte Abwärtsbewegung der Tröpfchen in der Kammer gezielt verzögert wird, so dass die in den Tröpfchen enthaltenen Mikroalgen während ihres Aufenthalts in der Kammer länger dem Licht und den in die Kammer eingebrachten Nährstoffen, wie insbesondere CO₂ ausgesetzt sind. In der Druckschrift wird außerdem die Möglichkeit angesprochen, das Licht, welchem die Mikroalgen ausgesetzt werden, gegebenenfalls künstlich zu erzeugen.

Unabhängig von dem jeweils genutzten Prinzip haben Einrichtungen zur Kultivierung von Mikroalgen mittels Bioreaktoren der zuvor beschriebenen Formen typischerweise einen sehr hohen Flächenbedarf - jedenfalls dann, wenn sie der Erzeugung größerer Mengen Biomasse dienen - oder sind, wie im Falle der in der DE 10 2009 028 474 A1 beschriebenen Ausbildungsform, nur für die Produktion kleinerer Mengen von Mikroalgenbiomasse ausgelegt. Zudem werden Bioreaktoren üblicherweise zumindest überwiegend unter Nutzung von Tageslicht beziehungsweise natürlichem Sonnenlicht betrieben. Sehr häufig werden daher entsprechende Einrichtungen zur Erzeugung größerer Mengen von Mikroalgenbiomasse auch in klimatisch günstig gelegenen, dünn besiedelten und daher große Flächen zur Verfügung stellenden Regionen errichtet. Dies ändert aber nichts an der Tatsache, dass entsprechende Einrichtungen wenig flächeneffizient und jahreszeitlich bedingten Schwankungen des jeweils zur Verfügung stehenden natürlichen Lichts unterworfen sind.

Aufgabe der Erfindung ist es, eine Lösung bereitzustellen, welche eine jahreszeitunabhängige und sehr flächeneffiziente Großproduktion von Mikroalgenbiomasse ermöglicht. Hierzu sind ein Verfahren und eine zur Durchführung des Verfahrens geeignete Einrichtung zur Produktion von Mikroalgenbiomasse anzugeben.

Die Aufgabe wird durch ein Verfahren mit den Merkmalen des Patentanspruchs 1 gelöst. Eine die Aufgabe lösende und zur Durchführung des Verfahrens geeignete Einrichtung für die Produktion von Mikroalgenbiomasse wird durch den ersten unabhängigen Sachanspruch charakterisiert. Vorteilhafte Aus- beziehungsweise Weiterbildungen der Erfindung sind durch die jeweiligen Unteransprüche gegeben.

Das vorgeschlagene Verfahren zur Produktion von Mikroalgenbiomasse geht von einem Ansatz aus, bei dem in einer Suspension aus Wasser und Mikroalgen enthaltene Mikroalgen in einem von der Suspension mehrfach passierten Kultivierungsmodul unter Zufuhr von Licht und von Nährstoffen, einschließlich CO₂ als gasförmigen Nährstoff, in einem kontinuierlichen Umlauf phototroph oder mixotroph kultiviert werden. Allerdings wird für das nachfolgend näher beschriebene Verfahren ausschließlich Licht mindestens einer künstlichen Lichtquelle verwendet.

Wiederholt werden Volumenanteile der im Umlauf geführten Suspension zur Ernte von Mikroalgen aus dem Kultivierungsmodul ausgeschleust sowie mittels einer Zentrifuge ausgeschleudert. Die nicht zur Ernte der Mikroalgen ausgeschleusten Volumenteile der Suspension verbleiben in dem durch einen Gasteil und einen Flüssigkeitsteil mit einem Flüssigkeitsvorrat ausgebildeten Kultivierungsmodul, indem sie aus dem Flüssigkeitsvorrat erneut dem Gasteil des Kultivierungsmoduls zugeführt und dort durch Zerstäuben ausgebracht werden. Das Ausschleusen von Volumenteilen der im Umlauf geführten Suspension aus dem Kultivierungsmodul zum Zweck der Ernte von Mikroalgen erfolgt jeweils, sofern die mittels optischer Sensoren festgestellte Trübung der Suspension einen Mindestwert überschreitet.

Für den insoweit festzulegenden Mindestwert der Trübung lässt sich allerdings keine feste Vorgabe machen. Nicht zuletzt sollte dieser Mindestwert sicherlich auch von der Art der jeweils zu kultivierenden Mikroalgen abhängen. Er sollte jedoch nicht zu gering festgelegt werden, da andernfalls aus den jeweils ausgeschleusten Volumenteilen der Suspension nur eine verhältnismäßig geringe Menge von Mikroalgen ausgeschleudert werden kann und der Erntevorgang dann möglicherweise wenig effizient ist. Dies ist aber letztlich im Grunde eine Optimierungsaufgabe im Zusammenhang mit dem Konfigurieren und Einstellen einer entsprechenden Einrichtung im Hinblick auf das Kultivieren einer bestimmten Mikroalgenart.

Bei dem zur Lösung der Aufgabe vorgeschlagenen Verfahren erfolgt die Kultivierung der Mikroalgen in einem als Klimakammer ausgebildeten Kultivierungsmodul. Die vorgenannte Klimakammer wird wasserwirtschaftlich betrieben. Bei dieser wasserwirtschaftlichen Betriebsweise erfolgt neben einer Regulierung der Temperatur der Suspension eine Regulierung ihres pH-Werts durch die gesteuerte Zugabe von Pufferionen sowie eine Regulierung des Redoxpotentials der Suspension und damit ihrer mikrobiellen Kontamination durch eine Steuerung der Licht- und Nährstoffzufuhr sowie der Zudosierung von Sauerstoff. Erfindungsgemäß wird ferner die nach dem Ausschleudern von Mikroalgen aus den ausgeschleusten Volumenteilen verbleibende Suspension mit einem hohen Wasseranteil und einem nur noch sehr geringen Anteil von Mikroalgen in das Kultivierungsmodul zurückgeführt, wobei sie vor ihrer Rückführung in das Kultivierungsmodul zur Abtötung unerwünschter mikrobieller Kontaminationen bis zum Erreichen eines minimalen in diesen Volumenteilen der Suspension gemessenen Redoxpotentials mit UV-Licht bestrahlt wird. Die Verweildauer der in das Kultivierungsmodul zurückzuführenden Suspension im Bereich einer zu ihrer Bestrahlung dienenden UV-Lichtquelle ist dabei umgekehrt proportional zu dem bei einer wiederholten Messung jeweils festgestellten Redoxpotential der Suspension. Je höher also das Redoxpotential ist, desto mehr verkürzt sich die Zeit, für welche die Suspension (nach dem Ausschleudern überwiegend nur noch Wasser) dem UV-Licht noch ausgesetzt werden muss.

In Ausgestaltung des zuvor grundsätzlich dargestellten erfindungsgemäßen Verfahrens kann sich dabei ein Ablauf vollziehen, entsprechend welchem mehrfach folgende Verfahrensschritte durchlaufen werden:
A. Versprühen zu kultivierende Mikroalgen enthaltender, aus dem Flüssigkeitsvorrat entnommener Volumenteile der Suspension im oberen Bereich des Gasteils des Kultivierungsmoduls, in welches Nährstoffe, darunter zumindest CO₂ als gasförmiger Nährstoff, eingeleitet werden.
B. Verzögern der schwerkraftbedingten Abwärtsbewegung beim Versprühen entstehender, die Mikroalgen enthaltender Tröpfchen der Suspension mittels geeigneter, dazu in dem Gasteil des Kultivierungsmoduls angeordneter Strukturelemente. Zur Art der vorgenannten Strukturelemente sollen nähere Ausführungen noch im Zusammenhang mit der Erläuterung der vorgeschlagenen, zur Durchführung des erfindungsgemäßen Verfahrens geeigneten Einrichtung erfolgen.
C. Exposition der in den in ihrer Abwärtsbewegung verzögerten Tröpfchen enthaltenen Mikroalgen mit dem hinsichtlich seiner Wellenlänge und seiner Intensität auf die Art der zu kultivierenden Mikroalgen abgestimmten Licht in dem Gasteil des Kultivierungsmoduls angeordneter LED-Lichtquellen und mit dem in das Kultivierungsmodul eingeleiteten CO₂. Dabei wird die Intensität des von den LED-Lichtquellen abgegebenen Lichts mit dem Fortschreiten des Kultivierungsvorganges verändert, nämlich mit zunehmender Trübung der Suspension erhöht.
D. Zurückführen sich abwärts bewegender, Mikroalgen enthaltender Tröpfchen in den am Boden oder in einem unterhalb des Gasteils des Kultivierungsmoduls angeordneten Behälter ausgebildeten Flüssigkeitsvorrat nach dem Passieren des Gasteils des Kultivierungsmoduls.
E. Ausschleusen von Volumenteilen von aus dem Flüssigkeitsvorrat zum erneuten Versprühen in dem Gasteil des Kultivierungsmoduls zurückgeführter Suspension sowie Ernte in den ausgeschleusten Volumenteilen enthaltener Mikroalgen durch Ausschleudern in einer Zentrifuge. Dabei erfolgt das Ausschleusen entsprechender Volumenteile der Suspension zum Zweck der Ernte von Mikroalgen, sofern die mittels optischer Sensoren festgestellte Trübung der dem Gasteil des Kultivierungsmoduls zum erneuten Versprühen gemäß dem ersten Verfahrensschritt (Verfahrensschritt A.) wieder zugeführter Volumenteile der Suspension einen Mindestwert überschreitet. Neben den dem Gasteil des Kultivierungsmoduls erneut zugeführten Volumenteilen der Suspension wird außerdem die nach dem Ausschleudern der ausgeschleusten Volumenteile verbleibende Suspension wieder in den Gasteil des Kultivierungsmoduls zurückgeführt, wobei diese Volumenteile der Suspension zuvor mit UV-Licht bestrahlt werden.

Im Zusammenhang mit dem gemäß den zuvor erläuterten Verfahrensschritten ablaufenden Verfahren wird gewissermaßen permanent der pH-Wert der Suspension in dem Flüssigkeitsteil des Kultivierungsmoduls gemessen und durch eine gesteuerte Zudosierung von Pufferionen reguliert. Der pH-Wert wird dabei in einem das Wachstum der Mikroalgen begünstigenden Bereich zwischen 7 und 8, vorzugsweise zwischen 7 und 7,5, gehalten. Zudem wird die mikrobielle Kontamination der Suspension durch eine in dem Flüssigkeitsteil des Kultivierungsmoduls (Klimakammer) durch periodische Messung erfolgende Überwachung ihres Redoxpotentials reguliert. Die Regulierung erfolgt, indem im Falle des Überschreitens eines oberen Grenzwerts für das Redoxpotential die Nährstoffzufuhr und die Lichtintensität der mindestens einen LED-Lichtquelle erhöht und im Falle des Unterschreitens eines unteren Grenzwertes für das Redoxpotential die Nährstoffzufuhr und die Lichtintensität der mindestens einen LED-Lichtquelle reduziert werden sowie die in dem Flüssigleitsteil des Kultivierungsmoduls erfolgende Zudosierung von Sauerstoff erhöht wird.

Die Lichtintensität der LED-Lichtquellen wird währenddessen, wie eingangs bereits ausgeführt, zusätzlich in Abhängigkeit von der gemäß dem 5. Verfahrensschritt (Verfahrensschritt E.) bestimmten Trübung der Suspension in der Weise gesteuert, dass die Lichtintensität proportional zur Trübung eingestellt wird.

Soweit vorstehend und in den Patentansprüchen im Zusammenhang mit dem Ausschleusen von Suspension aus dem Umlauf (Kreislauf) beziehungsweise aus dem Flüssigkeitsteil des Kultivierungsmoduls sowie mit der erneuten Zuführung der Suspension zum Gasteil des Kultivierungsmoduls wiederholt von Volumenteilen der Suspension die Rede ist, soll dies zum Ausdruck bringen, dass es sich jeweils nicht um die gesamte Suspension, sondern nur um einen Teil davon handelt.

Wie groß der jeweilige Volumenteil, insbesondere der aus dem Flüssigkeitsteil des Kultivierungsmoduls zur Ernte der Mikroalgen jeweils ausgeschleuste Volumenteil der Suspension vorzugsweise ist oder sinnvollerweise sein sollte, wäre im Zusammenhang mit der Implementierung des Verfahrens im Ergebnis entsprechender Testläufe festzulegen. Bei Berechnungen und Simulationen sowie im Ergebnis im Labormaßstab durchgeführter Tests hat es sich gezeigt, dass es vorteilhaft sein könnte, ein Volumenteil von 15 bis 50% des Volumens der sich in dem Flüssigkeitsteil des Kultivierungsmoduls befindenden Suspension auszuschleusen.

Die vorstehenden Erläuterungen und das Vorhandensein eines Vorratsbehälters lassen zudem erkennen, dass sich nie gleichzeitig die gesamte Menge der Suspension im Umlauf des von dem Gasteil und dem Flüssigkeitsteil des Kultivierungsmoduls gebildeten Kreislaufsystems befindet. Vielmehr befinden sich gewisse Volumenteile der Suspension zumindest vorübergehend in dem Flüssigkeitsvorrat und somit zumindest kurzzeitig gewissermaßen (nicht aus biologischer Sicht) in Ruhe.

Zur Regulierung des pH-Werts der Suspension, also zur Einstellung eines pH-Werts von vorzugsweise 7 bis 7,5, kann beispielsweise eine Zudosierung von Calcium- und/oder Magnesiumionen als Pufferionen erfolgen, wobei die Zudosierung dieser Ionen in dem Flüssigkeitsteil des Kultivierungsmoduls erfolgt. Zur Frage der Unterteilung der Klimakammer in einen Gasteil und in einen Flüssigkeitsteil sollen im Zusammenhang mit der Darstellung der zur Durchführung des Verfahrens geeigneten Einrichtungen noch einige Ausführungen erfolgen.

Entsprechend einer vorteilhaften Weiterbildung des Verfahrens kann zur Temperierung der Suspension die Flächentemperatur der in dem Kultivierungsmodul angeordneten LED-Lichtquellen genutzt werden, wobei diese wiederum durch eine Verringerung oder Erhöhung des Volumenstroms eines zur Kühlung der LED-Lichtquellen verwendeten Kühlmediums reguliert werden kann. Neben dem schon mehrfach erwähnten CO₂ als gasförmigem Nährstoff werden der Suspension in dem Flüssigkeitsteil des Kultivierungsmoduls typischerweise noch Stickstoff (zum Beispiel in Form von Ammoniak) und/oder Phosphor und/oder Kohlenstoff (zum Beispiel aus Kohlenstoffquellen wie Glukose) als Nährstoffe zugeführt.

Ferner kann das Verfahren so geführt werden, dass in den Mikroalgen gezielt bestimmte Sekundärinhaltsstoffe gebildet oder angereichert werden. Dazu können der Suspension in dem Flüssigkeitsteil des Kultivierungsmoduls, unter Einbeziehung geeigneter Stressfaktoren bei der Kultivierung, in gesteuerter Menge Stoffe oder Stoffgruppen einer oder mehrerer der folgenden zugeführt werden:
- Nährstoffe und mikrobiologische Kontaminationen zur Bildung von Vitaminen,
- Nährstoffe zur Bildung von Omega-3-Fettsäuren,
- Nährstoffe zur Bildung bioaktiver Proteine/Peptide,
- durch die Mikroalgen zu bindende Mineralien,
- durch die Mikroalgen zu bindendes Zink oder Eisen.

Das erfindungsgemäße Verfahren ermöglicht eine sehr flächeneffiziente Produktion großer Mengen von Algenbiomasse. Durch die Verwendung einer wasserwirtschaftlich betriebenen Klimakammer als Kultivierungsmodul werden ein sehr starkes und robustes Wachstum der Mikroalgen erreicht sowie die Erzeugung großer Mengen von Mikroalgenbiomasse mit einer insbesondere unter dem Gesichtspunkt möglicher mikrobieller Kontaminationen sehr hohen und gleichbleibenden Qualität ermöglicht, wobei es das Verfahrensregime mit seiner punktgenauen Einstellbarkeit wesentlicher Prozessparameter außerdem in hervorragender Weise ermöglicht, die Biomasse mit ausgewählten Sekundärstoffen, wie Vitaminen und/oder Mineralien in genau festgelegter Konzentration anzureichern.

Das Verfahren ermöglicht die Herstellung von Mikroalgenbiomasse kontinuierlich standort- und jahreszeitunabhängig, das heißt ganzjährig, mit gleicher Qualität hinsichtlich der Zusammensetzung von primären und sekundären Inhaltstoffen mit einer höheren Konzentration an Algensuspension im Wasser pro Liter im Vergleich zu den etablierten Verfahren (Open Pond, Photobioreaktor PBR und vergleichbaren Technologien) zur Mikroalgenbiomasseproduktion.

Eine zur Lösung der Aufgabe vorgeschlagene und zur Durchführung des Verfahrens geeignete Einrichtung zur Produktion von Mikroalgenbiomasse weist mindestens auf:
- wenigstens ein Kultivierungsmodul zur phototrophen oder mixotrophen Kultivierung in einer Suspension mit Wasser enthaltener Mikroalgen, wobei dieses Kultivierungsmodul aus einem Gasteil und einem Flüssigkeitsteil mit einem Flüssigkeitsvorrat besteht,
- Einleitungen und Ausbringungsorgane zur Einleitung von CO₂, sonstigen Nährstoffen und von Sauerstoff in das Kultivierungsmodul,
- wenigstens eine Zentrifuge zum Ausschleudern von Mikroalgen aus zum Zweck der Ernte aus dem wenigstens einen Kultivierungsmodul (Klimakammer) ausgeschleusten Volumenteilen der Suspension,
- ein Rohrleitungssystem mit Pumpen zur Bewegung der Suspension zwischen den vorgenannten Komponenten und zur Zuführung in das Kultivierungsmodul über die jeweiligen Ausbringungsorgane einzuleitender Stoffe, einschließlich von Nährstoffen und von Volumenteilen der Suspension,
- eine Steuereinrichtung zur Steuerung der wenigstens einen Zentrifuge sowie der vorgenannten Pumpen und Ausbringungsorgane entsprechend den Ergebnissen der Auswertung von ihr von Sensoren in den vorgenannten Komponenten empfangener Sensorsignale.

Dabei ist der Gasteil des wenigstens einen Kultivierungsmoduls mit mindestens einer in dessen oberen Bereich zur Ausbringung von Suspension angeordneten Düse, mit mindestens einer künstliches Licht emittierenden Lichtquelle, vorzugsweise mit mehreren LED-Lichtquelle(n), sowie mit Strukturelementen zur Verzögerung der schwerkraftbedingten Abwärtsbewegung beim Versprühen der Suspension entstehender Tröpfchen ausgestattet. Das Licht der vorgenannten mindestens einen Lichtquelle ist bezüglich seiner Wellenlänge und Intensität auf die Art der zu kultivierenden Mikroalgen abgestimmt oder abstimmbar.

Die Unterscheidung zwischen einem Gasteil und einem Flüssigkeitsteil des wenigstens einen Kultivierungsmoduls basiert darauf, dass die Suspension aus Mikroalgen und Wasser in dem als Flüssigkeitsteil betrachteten Teil des Kultivierungsmoduls tatsächlich als eine Flüssigkeit im eigentlichen Sinne vorliegt, wohingegen in dem Gasteil die Suspension aufgrund ihrer mittels in diesem Gasteil angeordneter Düsen erfolgenden Zerstäubung als ein feinzerstäubtes Aerosol und CO₂ als gasförmiger Nährstoff vorliegen. Insoweit wird der Flüssigkeitsteil des Kultivierungsmoduls durch den bereits genannten Flüssigkeitsvorrat (am Boden des Gasteils oder in einem Behälter unterhalb des Gasteils) sowie durch diesen Flüssigkeitsvorrat mit der Düse oder den Düsen im oberen Bereich des Gasteils verbindende Rohre des vorstehend bereits angesprochenen Rohrleitungssystems mit darin angeordneten Pumpen und Ventilen sowie mit gegebenenfalls weiteren in das Rohrleitungssystem einbezogenen Komponenten, wie Zwischentanks und dergleichen, ausgebildet. Nach diesem Verständnis gehören wiederum nicht alle Teile des Rohrleitungssystems zu dem Flüssigkeitsteil beziehungsweise zu dem eigentlichen Kultivierungsmodul, wie etwa das Kultivierungsmodul mit der Zentrifuge verbindende Rohrabschnitte und die darin angeordneten Komponenten.

Ganz bewusst wird auch im Zusammenhang mit dem Flüssigkeitsteil von einem Flüssigkeitsvorrat und nicht von einem Suspensionsvorrat gesprochen. Grund hierfür ist, dass bei einem Kultivierungsmodul der erfindungsgemäßen Einrichtung im Zusammenhang mit dessen erster Inbetriebnahme oder seiner Inbetriebnahme nach einer Umrüstung zunächst eine so genannte "Wasserfahrt" unternommen wird, bei welcher zunächst reines Wasser in einem ein- oder mehrmaligen Umlauf durch das Kultivierungsmodul mit dessen zu diesem Zeitpunkt noch mit Wasser gefüllten Flüssigkeitsvorrat in dem Flüssigkeitsteil geführt und das Wasser erst dann zur Bildung der später durch den Flüssigkeitsvorrat bevorrateten Suspension mit Mikroalgen beimpft wird. Hierbei sei angemerkt, dass gegebenenfalls aber auch unmittelbar eine Suspension mit bereits "vorkultivierten Mikroalgen in das Kultivierungsmodul eingebracht werden oder zunächst eingebrachtes Wasser durch Zufuhr von Suspension mit "vorkultivierten Mikroalgen beimpft werden kann. Das wenigstens eine Kultivierungsmodul der erfindungsgemäßen Einrichtung weist vorzugsweise eine Größe mit einer Grundfläche von mindestens 250 m² auf.

Erfindungsgemäß ist das wenigstens eine Kultivierungsmodul als eine Klimakammer ausgebildet, welche entsprechend dem Verfahren wasserwirtschaftlich betrieben wird. Selbstverständlich wird, gesteuert von der Steuereinrichtung, die Temperatur der mehrfach durch das Kultivierungsmodul geleiteten Suspension reguliert. Die für die Suspension jeweils einzustellende Temperatur hängt dabei von der Art der jeweils zu kultivierenden Mikroalgen ab. Dabei liegt es auf der Hand, dass beispielsweise entsprechend ihrem natürlichen Vorkommen im Polarmeer beheimatete Mikroalgen für eine erfolgreiche Kultivierung deutlich niedrigere Temperaturen benötigen als beispielsweise Mikroalgenarten, wie sie natürlicherweise in Gewässern europäischer Regionen mit einem gemäßigten Klima vorkommen.

Für den wasserwirtschaftlichen Betrieb des wenigstens einen Kultivierungsmoduls sind sowohl in dessen Gasteil als auch in dessen Flüssigkeitsteil mehrere mit der Steuereinrichtung in Wirkverbindung stehende Sensoren und Ausbringungsorgane angeordnet. Im Einzelnen handelt es sich hierbei zum einen insbesondere um in dem Flüssigkeitsteil angeordnete Sensoren (Elektroden) zur Bestimmung des pH-Werts der Suspension und um Ausbringungsorgane, welche durch die Steuereinrichtung entsprechend dem mittels der vorgenannten Sensoren jeweils festgestellten pH-Wert für eine gesteuerte Zugabe von Pufferionen in dem Vorratsbehälter angesteuert werden. Ferner sind in dem Flüssigkeitsteil des Kultivierungsmoduls (Klimakammer) Sensoren zur wiederholten Messung des Redoxpotentials der Suspension angeordnet, deren Sensorsignale durch die Steuerungseinrichtung zur Regulierung des Redoxpotentials durch eine gesteuerte Zufuhr von Nährstoffen, einschließlich CO₂, und von Sauerstoff sowie zur Steuerung der Lichtintensität des von den vorzugsweise mehreren Lichtquellen (besonders bevorzugt LED-Lichtquellen) emittierten Lichtes verarbeitet werden.

Komplettiert werden die für den wasserwirtschaftlichen Betrieb der Klimakammer (des in einem wasserwirtschaftlichen Betrieb klimatisierten Kultivierungsmoduls) erforderlichen Komponenten durch Sensoren zur wiederholten Messung des Redoxpotentials der dem Gasteil des Kultivierungsmoduls erneut zugeführten Suspension mit sehr hohem Wasseranteil, welche beim Ausschleudern der zur Ernte ausgeschleusten Volumenanteile zurückbleibt, sowie durch eine UV-Lichtquelle. Die vorgenannten Sensoren und die UV-Lichtquelle sind dabei vorzugsweise in einer Rohrleitung zur Rückführung dieser Volumenteile der Suspension in das Kultivierungsmodul angeordnet. Mittels dieser Sensoren wird, wie bereits ausgeführt, das Redoxpotential der nach dem Ausschleudern in das Kultivierungsmodul zurückzuführenden Suspension mit hohem Wasseranteil und wenigen darin gegebenenfalls noch verbliebenen Mikroalgen wiederholt gemessen und die zurückzuführende Suspension, gesteuert durch die Steuereinrichtung, bis zum Erreichen eines minimalen Redoxpotentials mit UV-Licht bestrahlt.

Durchgeführten Berechnungen zufolge kann mittels eines Kultivierungsmoduls der erfindungsgemäßen Einrichtung auf einer Fläche von etwa 250 m² eine Menge von Mikroalgenbiomasse erzeugt werden, für deren Erzeugung mittels eines Rohr-Bioreaktors eine Rohrlänge von etwa 500 km erforderlich wäre. Im Zusammenhang mit einer praktischen Umsetzung der vorgeschlagenen Lösung könnten beispielsweise Rohrreaktoren kleiner bis mittlerer Größe als Rohstofflieferanten gelten, indem Mikroalgen in ihnen gewissermaßen "vorkultiviert" und dann, als Bestandteil einer Suspension, zum Zweck einer intensiven Kultivierung unter insbesondere für die wasserwirtschaftliche betriebene Klimakammer exakt einstellbaren Verfahrensbedingungen, einem Kultivierungsmodul einer erfindungsgemäß ausgebildeten Einrichtung zugeführt werden.

Bei den in dem Gasteil eines jeweiligen Kultivierungsmoduls zur Verzögerung der Abwärtsbewegung der Mikroalgen enthaltenden Tröpfchen angeordneten Strukturelementen kann es sich beispielsweise um horizontal angeordnete Flächenelemente in Form straff gespannter textiler Vliese oder feinmaschiger textiler Netze handeln. Die betreffenden Strukturelemente werden vorzugsweise sehr straff gespannt, um zu vermeiden, dass sie aufgrund der Benetzung mit der Suspension stark durchhängen und sie dadurch verschattete Bereiche innerhalb des Gasteils des Kultivierungsmoduls erzeugen.

Für eine besonders gute und homogene Durchflutung des Gasteils des Kultivierungsmoduls mit dem von der mindestens einen, vorzugsweise von den mehreren LED-Lichtquellen abgegebenen Licht, sind bei einer besonders vorteilhaften Ausbildungsform der erfindungsgemäßen Einrichtung die Wände des Kultivierungsmoduls in dem Gasteil (gegebenenfalls voll) verspiegelt oder auf ihrer Innenseite hochglanz-reflektierend ausgebildet. Letzteres heißt, dass die Wände aus einem reflektierenden Hochglanzmaterial ausgebildet oder mit einem solchen Material (gegebenenfalls vollflächig) beschichtet sein können. Hierdurch ist eine besonders effiziente Ausnutzung der in das Kultivierungsmodul eingebrachten Lichtenergie gegeben.

Vorteilhafterweise kann es außerdem vorgesehen sein, dass die Temperierung der Suspension unter Ausnutzung der Abwärme der LED-Lichtquellen (nachfolgend wird vom Vorhandensein mehrerer Lichtquellen ausgegangen) erfolgt. Hierzu wird ein zur Kühlung der LED-Lichtquellen verwendetes Kühlmedium hinsichtlich seines Volumenstroms entsprechend reguliert, so dass die Flächentemperatur einer jeden LED-Lichtquelle ausgehend von ihrem jeweiligen Beitrag für den Wärmeeintrag in die Klimakammer entsprechend der jeweils für die Suspension benötigten Temperatur mit Hilfe der Steuereinrichtung eingestellt werden kann. Anders als bei bisher bekannt gewordenen Gewächshäusern oder Einrichtungen zur Kultivierung von Mikroorganismen kann hierdurch der Einfluss von durch Lichtquellen in ein derartiges System eingebrachter Wärme gezielt kontrolliert werden.

Das ist bei bisher bekannt gewordenen Systemen nicht der Fall, da bei diesen die durch eventuelle künstliche Lichtquellen eingebrachte Wärmemenge regelmäßig weder bekannt noch kontrollierbar ist. Es ist aber andererseits keineswegs so, dass im Falle des Erfordernisses einer höheren Lichtintensität zwingend auch eine höhere Temperatur in dem jeweiligen System (Kultivierungseinrichtung, Gewächshaus) benötigt wird.

Für die Großproduktion von Mikroalgenbiomasse umfasst eine in erfindungsgemäßer Weise ausgebildete Einrichtung vorzugsweise eine Mehrzahl von Kultivierungsmodulen und mindestens eine, gegebenenfalls aber auch mehrere, jeweils durch mehrere Kultivierungsmodule gemeinsam genutzte Zentrifugen für das Ausschleudern der Biomasse zum Zweck ihrer Ernte. Ein solcher modularer Aufbau bringt dabei den Vorteil mit sich, dass im Falle dessen, dass beispielsweise in einem Kultivierungsmodul der Kultivierungsprozess der Mikroalgen nicht in gewünschter Weise verläuft oder eventuell unerwünschte mikrobielle Kontaminierungen auftreten, nur die innerhalb dieser einen Kultivierungsmoduls im Umlauf geführte Suspension verworfen werden muss, wohingegen aus der Suspension anderer Kultivierungsmodule der Einrichtung weiterhin Mikroalgen mit der jeweils geforderten Beschaffenheit und Qualität geerntet werden können. Im Gegensatz zu allen vergleichbaren und im Wettbewerb um Produktivität stehenden Systemen handelt es sich bei dem oder einem Kultivierungsmodul der erfindungsgemäßen Einrichtung um ein kontrolliertes, geschlossenes System. Die Gefahr von unerwünschten Kontamination, wie sie beispielsweise bei Open Pond Anlagen existiert, kann hierdurch nahezu ausgeschlossen werden.

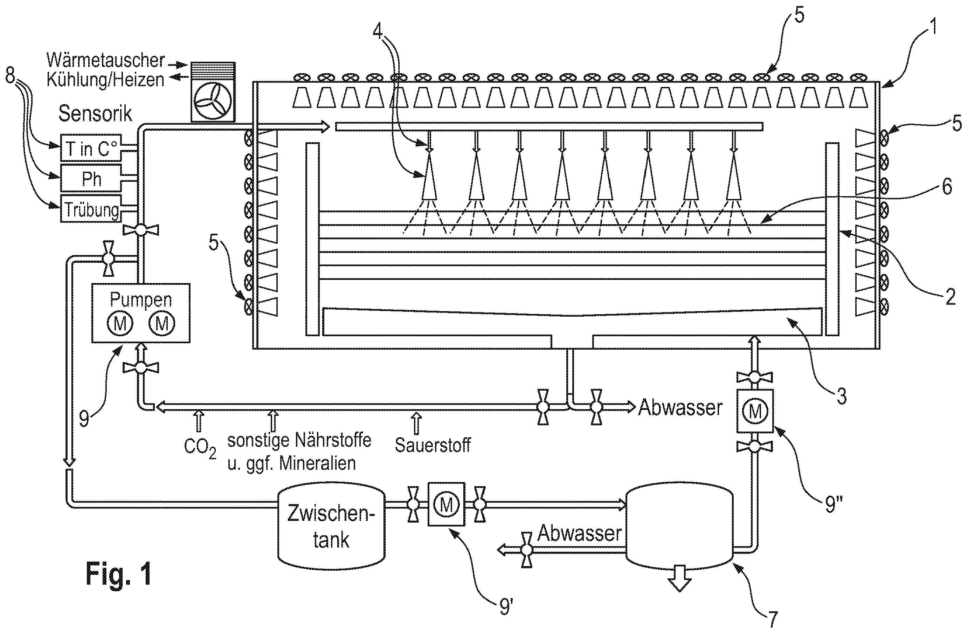

Anhand der Fig. 1 sollen nachfolgend einige Aspekte der Erfindung nochmals näher erläutert werden.

Die Fig. 1 zeigt beispielhaft eine mögliche Ausbildungsform eines Kultivierungsmoduls der erfindungsgemäßen Einrichtung einschließlich einer mit ihm gekoppelten Zentrifuge. Das Kultivierungsmodul bildet einen zentralen beziehungsweise den zentralen Bestandteil der erfindungsgemäßen Einrichtung zur Produktion von Mikroalgenbiomasse, auf welchem letztlich auch der Fokus der Erfindung liegt, so dass auf die Darstellung vieler übriger Bestandteile der Einrichtung (zum Beispiel Steuerung[en] und dergleichen) verzichtet wurde. Je nach Auslegung einer erfindungsgemäß gestalteten Einrichtung kann diese eine größere Zahl derartiger Kultivierungsmodule aufweisen. In einem solchen Fall arbeiten mehrere dieser Kultivierungsmodule mit einer der Ernte der Mikroalgen durch Ausschleudern aus der Suspension dienenden (hier nicht dargestellten) Zentrifuge zusammen. In sehr großen Anlagen können gegebenenfalls auch mehrere Zentrifugen vorhanden sein, wobei aber typischerweise nicht jedem Kultivierungsmodul separat eine Zentrifuge zugeordnet werden, sondern jede der Zentrifugen jeweils mit mehreren Kultivierungsmodulen zusammenarbeiten wird.

Das in der Fig. 1 beispielhaft gezeigte Kultivierungsmodul 1, von dem, wie gesagt, gegebenenfalls mehrere in einer erfindungsgemäß ausgebildeten Einrichtung vorhanden sein können, besteht aus dem Gasteil 2 und dem Flüssigkeitsteil, welcher einen Flüssigkeitsvorrat 3 zur Aufnahme eines Vorrats einer aus Wasser und den zu kultivierenden Mikroalgen bestehenden Suspension umfasst. Der Flüssigkeitsvorrat 3 ist, wie aus der Fig. 1 ersichtlich, am Boden des Gasteils 2 des Kultivierungsmoduls 1 angeordnet. In einem, abgesehen von eventuellen Umrüstzeiten, kontinuierlichen Vorgang beziehungsweise Kreisprozess werden Volumenteile der Suspension aus dem Flüssigkeitsvorrat 3 dem Gasteil 2 des als wasserwirtschaftlich betriebene Klimakammer ausgebildeten Kultivierungsmoduls 1 zugeleitet. Dies geschieht über zum Flüssigkeitsteil des Kultivierungsmodul 1 gehöhrende Rohre eines Rohrleitungssystems mit Hilfe der Pumpe(n) 9.

Die der Klimakammer, also dem in einer wasserwirtschaftlichen Betriebsweise klimatisierten Kultivierungsmodul 1 zugeleitete Suspension wird in dem oberen Bereich des Gasteils 2 - also vorzugsweise unmittelbar unterhalb der Raumdecke - mit Hilfe mehrerer Düsen 4 durch Versprühen ausgebracht. In dem Gasteil 2 des Kultivierungsmoduls 1 sind mehrere bezüglich der Wellenlänge und der Intensität des von ihnen ausgesendeten Lichtes steuerbare LED-Lichtquellen 5 - hier in Form eines oder mehrerer an der Decke und den Wänden des Gasteils 2 des Kultivierungsmoduls 1 installierter Lichtbänder - angeordnet. Außerdem verfügt das Kultivierungsmodul 1 über Einleitungen zur Zuführung von Nährstoffen, einschließlich CO₂ als gasförmigen Nährstoff, sowie von Sauerstoff für die in ihm kultivierten Mikroalgen.

Die in dem Gasteil 2 des Kultivierungsmoduls 1 über Düsen 4 versprühten Volumenteile der Suspension bilden infolge des Versprühens einen Nebel (Aerosol) aus jeweils Mikroalgen enthaltenden Tröpfchen aus. Die Tröpfchen bewegen sich schwebend allmählich zum Boden des Gasteils 2 des Kultivierungsmoduls 1, wobei in dem Gasteil 2 außerdem, durch textile Netze oder textile Vliese ausgebildete Strukturen 6 angeordnet sind, welche die schwerkraftbedingte Abwärtsbewegung der Tröpfchen verzögern. Zweck dieser Maßnahme ist es, die Aufenthaltsdauer der in dem Kultivierungsmodul 1 versprühten Volumenteile der Suspension, also der die Mikroalgen enthaltenden Tröpfchen, in dem Gasteil zu verlängern, damit diese zur Förderung des Algenwachstums und der Algenvermehrung möglichst lange der Exposition mit dem künstlichen Licht der LED-Lichtquellen 5 und mit dem in das Kultivierungsmodul 1 eingetragenen CO₂ ausgesetzt sind.

Die sich am Boden des Gasteils 2 des Kultivierungsmoduls 1 durch auftreffende Tröpfchen in dem Flüssigkeitsvorrat 3 ansammelnde Suspension wird von hier aus erneut dem Gasteil 2 des Kultivierungsmoduls 1 zugeleitet. Im Zuge des mehrfachen Umlaufens der Suspension in diesem Kreislauf erhöht sich durch das Algenwachstum allmählich die Trübung der Suspension. Mittels in den Rohrleitungen angeordneter optischer Sensoren 8 wird ständig die Trübung der wiederholt dem Gasteil 2 des Kultivierungsmoduls 1 zugeleiteten Suspension bestimmt. Hierzu weist die erfindungsgemäße Einrichtung eine mit den vorgenannten Sensoren 8 sowie mit weiteren Sensoren in einer Wirkverbindung stehende (nicht gezeigte) Steuereinrichtung auf.

Bei der Steuereinrichtung kann es sich um eine zentrale Steuereinheit oder um eine durch mehrere dezentral angeordnete, gemeinsam die Steuereinrichtung ausbildende Steuereinheiten handeln. Die Steuereinrichtung steht zudem in einer Wirkverbindung mit mehreren von ihr entsprechend den Ergebnissen der Auswertung von den Sensoren 8 empfangener Sensorsignale gesteuerten Aktoren, wie Ausbringungsorganen (hierzu zählen unter anderem die Düsen 4 in dem Gasteil 2 des Kultivierungsmoduls 1) und steuerbaren Ventilen. Überschreitet die Trübung der wiederholt dem Gasteil 2 des Kultivierungsmoduls 1 zugeführten Volumenteile der Suspension einen in der Steuereinrichtung durch entsprechende Konfiguration festgelegten Mindestwert, so wird, veranlasst durch die Steuereinrichtung, ein Teil (Volumenteil) der sich in diesem Moment in dem Flüssigkeitsteil befindenden Suspension aus dem Kultivierungsmodul 1 ausgeschleust und der Zentrifuge 7 zugeleitet.

In der Zentrifuge 7 werden die in den ausgeschleusten Volumenteilen der Suspension enthaltenen Mikroalgen ausgeschleudert und nachfolgenden, gegebenenfalls nicht mehr in der hier betrachteten Einrichtung durchgeführten Verarbeitungsvorgängen zugeführt. Die beim Ausschleudern der aus dem Kultivierungsmodul 1 ausgeschleusten Volumenteile zurückbleibende, ganz überwiegend aus Wasser bestehende Suspension wird wieder in das Kultivierungsmodul 1 zurückgeführt, wobei sie aber zuvor durch Bestrahlung mit UV-Licht zur Beseitigung mikrobieller Kontaminationen behandelt wird. Letzteres erfolgt in einem Abschnitt der Rohrverbindung, über welche diese Volumenteile der überwiegend aus Wasser bestehenden Suspension erneut dem Kultivierungsmodul 1 zugeführt werden. Die Verweildauer der nach dem Ausschleudern der aus dem Kultivierungsmodul 1 ausgeschleusten Volumenteile zurückbleibenden Suspension in dem mit einer entsprechenden (hier nicht gezeigten) UV-Lichtquelle ausgestatteten Rohrabschnitt hängt von der für die Abtötung eventueller mikrobieller Kontaminationen benötigten Zeit ab, wobei die Suspension bis zum Erreichen eines minimalen, mittels (ebenfalls nicht gezeigter) Sensoren (Elektroden) innerhalb des Rohrabschnitts zwischen dem Ausgang der Zentrifuge 7 und dem Kultivierungsmodul 1 festgestellten Redoxpotentials im Bereich des UV-Lichteintrages verbleibt.

Zur Realisierung der wasserwirtschaftlichen Betriebsweise der das Kultivierungsmodul 1 ausbildenden Klimakammer sind, zumindest in deren Flüssigkeitsteil, noch weitere Sensoren 8 angeordnet, welche in einer Wirkverbindung mit der (nicht gezeigten) Steuereinrichtung stehen. Es handelt sich hierbei zumindest um Sensoren 8 - beispielsweise in Form von Silberchloridelektroden - zur Bestimmung des pH-Werts der Suspension sowie um Sensoren 8 - ebenfalls spezielle Elektroden - zur Bestimmung des Redoxpotentials der Suspension. Entsprechend dem Ergebnis aus der fortwährenden Messung des pH-Werts der Suspension wird durch die Steuereinrichtung eine Zudosierung von Pufferionen, nämlich von Calciumionen und/oder Magnesiumionen, durch Betätigung entsprechender, dafür in dem Flüssigkeitsteil des Kultivierungsmoduls 1 angeordneter (hier ebenfalls nicht im Detail gezeigter) Ausbringungsorgane gesteuert.

Das Redoxpotential der Suspension wird, gesteuert durch die Steuereinrichtung, dadurch reguliert, dass im Falle des Unterschreitens eines Redoxpotentials von 100 mV ein Stopp der Nährstoffzufuhr (von in den Flüssigkeitsteil eingebrachten Nährstoffen sowie des letztlich im Gasteil 2 des Kultivierungsmoduls 1 ausgebrachten gasförmigen Nährstoffs CO₂) und eine Verringerung der Intensität des durch die LED-Lichtquellen 5 in dem Gasteil 2 des Kultivierungsmoduls 1 emittierten Lichts erfolgt, wobei gleichzeitig der Sauerstoffeintrag durch Ansteuerung entsprechender Ausbringungsorgane in dem Flüssigkeitsbereich erhöht wird. Im Falle eines zu hohen Redoxpotentials, nämlich eines Redoxpotentials von mehr als 300 mV, werden die Lichtintensität im Gasteil 2 des Kultivierungsmoduls 1 und der Eintrag von Nährstoffen, nämlich des in das Kultivierungsmodul 1 eingebrachten CO₂ und anderer eingeleiteter Nährstoffe, erhöht.

Um während des gesamten Kultivierungszyklus einen weitestgehend konstanten Energieeintrag in Form des von den LED-Lichtquellen 5 ausgesendeten Lichts zu bewerkstelligen, wird außerdem mit zunehmender, im Flüssigkeitsteil des Kultivierungsmoduls 1 sensorisch festgestellter Trübung der Suspension, die Lichtintensität im Gasteil 2 des Kultivierungsmoduls 1 erhöht. Zur Unterstützung der Temperierung der Suspension kann durch die Steuerung auf der Basis der mittels mindestens eines Temperatursensors in dem Flüssigkeitsteil des Kultivierungsmoduls 1 ermittelten Suspensionstemperatur der Volumenstrom eines durch aktive Kühlelemente für die LED-Lichtquellen 5 geleiteten Kühlmediums gesteuert und hierdurch die Flächentemperatur der nicht nur Licht, sondern (gewissermaßen als Nebenprodukt) auch Wärme in das Kultivierungsmodul 1 abgebenden LED-Lichtquellen 5 gesteuert werden, wobei gegebenenfalls die Temperierung der Suspension auch ausschließlich auf der Basis einer solchen Steuerung erfolgen kann.

## Patentansprüche

1. Verfahren zur Produktion von Mikroalgenbiomasse, bei dem in einer Suspension aus Wasser und Mikroalgen enthaltene Mikroalgen in einem von der Suspension mehrfach passierten, einen Gasteil (2) und einen Flüssigkeitsteil mit einem Flüssigkeitsvorrat (3) aufweisenden Kultivierungsmodul (1) unter Zufuhr von den gesamten Gasteil (2) des Kultivierungsmoduls (1) gleichmäßig durchflutendem Licht mindestens einer künstlichen Lichtquelle (5) und von Nährstoffen in einem kontinuierlichen Umlauf phototroph oder mixotroph kultiviert werden, wobei wiederholt Volumenanteile der Suspension zur Ernte von Mikroalgen aus dem Kultivierungsmodul (1) ausgeschleust sowie mittels einer Zentrifuge (7) ausgeschleudert werden und die nach dem Ausschleudern verbleibende Suspension wieder dem Kultivierungsmodul (1) zugeführt wird, **dadurch gekennzeichnet, dass** das Ausschleusen von Volumenteilen der Suspension zur Ernte von Mikroalgen jeweils erfolgt, sofern die mittels optischer Sensoren (8) festgestellte Trübung der Suspension einen Mindestwert überschreitet, und dass die Kultivierung der Mikroalgen in einer das Kultivierungsmodul (1) ausbildenden Klimakammer erfolgt, welche wasserwirtschaftlich betrieben wird, indem neben einer Regulierung der Temperatur der Suspension eine Regulierung ihres pH-Werts durch gesteuerte Zugabe von Pufferionen und eine Regulierung des Redoxpotentials der Suspension und damit ihrer mikrobiellen Kontamination durch eine Steuerung der Licht- und Nährstoffzufuhr sowie einer Zudosierung von Sauerstoff erfolgen und indem die nach dem Ausschleudern der Mikroalgen verbleibende Suspension vor ihrer Rückführung in das Kultivierungsmodul (1) zur Abtötung unerwünschter mikrobieller Kontaminationen bis zum Erreichen eines minimalen, in der Suspension gemessenen Redoxpotentials mit UV-Licht bestrahlt wird.

2. Verfahren nach Anspruch 1, **gekennzeichnet durch** die mehrfach durchlaufenen Verfahrensschritte:
a.) Versprühen zu kultivierende Mikroalgen enthaltender, aus dem Flüssigkeitsvorrat (3) des Flüssigteils entnommener Volumenteile der Suspension im oberen Bereich des Gasteils (2) des Kultivierungsmoduls (1), in welches Nährstoffe, darunter mindestens CO₂ als gasförmiger Nährstoff, eingeleitet werden,
b.) Verzögern der schwerkraftbedingten Abwärtsbewegung beim Versprühen entstehender, die Mikroalgen enthaltender Tröpfchen der Suspension mittels geeigneter, dazu in dem Gasteil (2) des Kultivierungsmoduls (1) angeordneter Strukturelemente (6),
c.) Exposition der in den in ihrer Abwärtsbewegung verzögerten Tröpfchen enthaltenen Mikroalgen mit dem hinsichtlich seiner Wellenlänge und seiner Intensität auf die Art der zu kultivierenden Mikroalgen abgestimmten Licht in dem Gasteil (2) des Kultivierungsmoduls (1) angeordneter LED-Lichtquellen (5) und mit dem eingeleiteten CO₂,
d.) Zurückführen sich abwärts bewegender, Mikroalgen enthaltender Tröpfchen in den am Boden oder in einem Behälter unterhalb des Gasteils (2) des Kultivierungsmoduls (1) ausgebildeten Flüssigkeitsvorrat (3) nach dem Passieren des Gasteils (2) des Kultivierungsmoduls (1),
e.) Ausschleusen von Volumenteilen von aus dem Flüssigkeitsvorrat (3) zum erneuten Versprühen in dem Gasteil (2) des Kultivierungsmoduls (1) zurückgeführter Suspension sowie Ernte in den ausgeschleusten Volumenteilen enthaltener Mikroalgen durch Ausschleudern in einer Zentrifuge (7), sofern die mittels optischer Sensoren (8) festgestellte Trübung der dem Gasteil (2) des Kultivierungsmoduls (1) zum erneuten Versprühen gemäß Verfahrensschritt a) wieder zugeführter Volumenteile der Suspension einen Mindestwert überschreitet, sowie Rückführung der nach dem Ausschleudern der ausgeschleusten Volumenteile verbleibenden Suspension in das Kultivierungsmodul (1), nach einer Bestrahlung mit UV-Licht,
wobei
- der pH-Wert der Suspension in dem Flüssigkeitsteil gemessen sowie durch eine gesteuerte Zudosierung von Pufferionen reguliert und dabei in einem das Wachstum der Mikroalgen begünstigenden pH-Wertbereich zwischen 7 und 8, vorzugsweise zwischen 7 und 7,5, gehalten wird,
- die mikrobielle Kontamination der Suspension durch eine Überwachung ihres Redoxpotentials reguliert wird, indem im Falle des Überschreitens eines oberen Grenzwertes für das Redoxpotential die Nährstoffzufuhr und die Lichtintensität der LED-Lichtquellen erhöht und im Falle des Unterschreitens eines unteren Grenzwertes für das Redoxpotential die Nährstoffzufuhr und die Lichtintensität der LED-Lichtquellen (5) reduziert werden sowie die in dem Flüssigkeitsteil des Kultivierungsmoduls (1) erfolgende Zudosierung von Sauerstoff erhöht wird, wobei die Lichtintensität der LED-Lichtquellen (5) zusätzlich in Abhängigkeit von der gemäß Verfahrensschritt e) bestimmten Trübung der Suspension in der Weise gesteuert wird, dass sie proportional zur Trübung eingestellt wird.

3. Verfahren nach Anspruch 2, **dadurch gekennzeichnet, dass** zur Temperierung der Suspension die Flächentemperatur der im Gasteil des Kultivierungsmoduls angeordneten LED-Lichtquellen (5) genutzt wird, welche durch eine Verringerung oder Erhöhung des Volumenstroms eines zur Kühlung der LED-Lichtquellen (5) verwendeten Kühlmediums reguliert wird.

4. Verfahren nach einem der Ansprüche 1 bis 3, **dadurch gekennzeichnet, dass** zur Regulierung des pH-Wertes der Suspension in dem Flüssigkeitsteil des Kultivierungsmoduls (1) Calcium- und/oder Magnesiumionen als Pufferionen für das Kohlensäuregleichgewicht zudosiert werden.

5. Verfahren nach einem der Ansprüche 1 bis 4, **dadurch gekennzeichnet, dass** der Suspension in dem Flüssigkeitsteil des Kultivierungsmoduls (1) Stickstoff, Phosphor und Kohlenstoff als Nährstoffe zugeführt werden.

6. Verfahren nach einem der Ansprüche 1 bis 5, **dadurch gekennzeichnet, dass** in den Mikroalgen Sekundärinhaltstoffe gebildet oder angereichert werden, indem der Suspension in dem Flüssigkeitsteil des Kultivierungsmoduls (1) Stoffe oder Stoffgruppen in gesteuerter Menge zugeführt werden, welche wenigstens einer der folgenden Kategorien angehören:
- Nährstoffe und mikrobiologische Kontaminationen zur Bildung von Vitaminen,
- Nährstoffe zur Bildung von Omega-3-Fettsäuren,
- Nährstoffe zur Bildung bioaktiver Proteine/Peptide,
- durch die Mikroalgen zu bindende Mineralien,
- durch die Mikroalgen zu bindendes Zink oder Eisen.

7. Verfahren nach einem der Ansprüche 1 bis 6, **dadurch gekennzeichnet, dass** im Falle des Überschreitens des für die Trübung der Suspension festgelegten Mindestwertes aus dem Kultivierungsmodul (1) Volumenanteile der Suspension in einer Größenordnung von 15% bis 50% des sich im Flüssigkeitsteil des Kultivierungsmoduls (1) befindenden Volumens der Suspension ausgeschleust werden.

8. Einrichtung zur Produktion von Mikroalgenbiomasse, wobei die Einrichtung mindestens aufweist
- wenigstens ein Kultivierungsmodul (1) zur phototrophen oder mixotrophen Kultivierung in einer Suspension mit Wasser enthaltener Mikroalgen, bestehend aus einem Gasteil (2) und einem Flüssigkeitsteil mit einem Flüssigkeitsvorrat (3) und mit mindestens einer als Ausbringungsorgan für Suspension in einem oberen Bereich des Gasteils angeordneten Düse (4), mit mindestens einer in dem Gasteil (2) angeordneten Lichtquelle (5) zur Abgabe hinsichtlich seiner Wellenlänge sowie seiner Intensität auf die Art der zu kultivierenden Mikroalgen abgestimmten künstlichen Lichts sowie mit in dem Gasteil (2) zur Verzögerung der schwerkraftbedingten Abwärtsbewegung beim Versprühen der Suspension entstehender Tröpfchen angeordneten Strukturelementen (6),
- Einleitungen und Ausbringungsorgane zur Einleitung von CO₂, sonstigen Nährstoffen und Sauerstoff in das wenigstens eine Kultivierungsmodul (1),
- wenigstens eine Zentrifuge (7) zum Ausschleudern von Mikroalgen aus Volumenteilen der Suspension, welche zum Zweck der Ernte der Mikroalgen aus dem wenigstens einen Kultivierungsmodul (1) ausgeschleust und der Zentrifuge (7) zugeführt werden,
- ein Rohrleitungssystem mit Pumpen (9, 9', 9") zur Bewegung der Suspension zwischen den vorgenannten Komponenten und zur Zuführung in das Kultivierungsmodul (1) über die jeweiligen Ausbringungsorgane einzuleitender Stoffe, einschließlich von Nährstoffen und von Volumenteilen der Suspension,
- eine Steuereinrichtung zur Steuerung der wenigstens einen Zentrifuge (7) sowie der vorgenannten Pumpen (9, 9', 9") und Ausbringungsorgane entsprechend den Ergebnissen der Auswertung von unterschiedlichen, in den Komponenten der Einrichtung angeordneten und mit der Steuerung in einer Wirkverbindung stehenden Sensoren (8) empfangenen Sensorsignalen,
**dadurch gekennzeichnet, dass**
das wenigstens eine Kultivierungsmodul (1) als eine Klimakammer ausgebildet ist, welche wasserwirtschaftlich betrieben wird, indem neben einer Regulierung der Temperatur der Suspension mittels der Steuereinrichtung auf der Grundlage von Sensorsignalen der in dem wenigstens einen Kultivierungsmodul (1) angeordneten Sensoren (8) eine Regulierung des pH-Werts der Suspension durch gesteuerte Zugabe von Pufferionen in den Flüssigkeitsteil des wenigstens einen Kultivierungsmoduls (1) und eine Regulierung des mittels Sensoren (8) wiederholt gemessenen Redoxpotentials der Suspension durch eine von der Steuereinrichtung gesteuerte Zufuhr des CO₂, der sonstigen Nährstoffe und von Sauerstoff in das wenigstens eine Kultivierungsmodul (1) sowie der Lichtintensität in dem Gasteil (2) des wenigstens einen Kultivierungsmoduls (1) erfolgen und indem beim Ausschleudern der Mikroalgen aus ausgeschleusten Volumenanteilen verbleibende, dem wenigstens einen Kultivierungsmodul (1) erneut zugeführte Suspension vor ihrer erneuten Einleitung in das wenigstens eine Kultivierungsmodul (1) zur Abtötung unerwünschter mikrobieller Kontaminationen gesteuert von der Steuereinrichtung bis zum Erreichen eines minimalen in der zurückzuführenden Suspension sensorisch gemessenen Redoxpotentials mit UV-Licht bestrahlt wird.

9. Einrichtung nach Anspruch 8, **dadurch gekennzeichnet, dass** der Gasteil (2) des wenigstens einen Kultivierungsmoduls (1) reflektierende Wände aufweist, die auf ihrer Innenseite verspiegelt oder hochglanz-reflektierend ausgebildet sind.

10. Einrichtung nach Anspruch 9, **dadurch gekennzeichnet, dass** die Seitenwände und die Decke des Gasteils (2) des wenigstens einen Kultivierungsmoduls (1) auf ihrer Innenseite vollverspiegelt oder vollflächig hochglanz-reflektierend ausgebildet sind.

11. Einrichtung nach Anspruch 8 oder 9, **dadurch gekennzeichnet, dass** die mindestens eine in dem Gasteil (2) des wenigstens einen Kultivierungsmoduls (1) angeordnete Lichtquelle (5) gleichzeitig als Heizung zur Temperierung der Suspension in diesem wenigstens einen Kultivierungsmodul (1) fungiert, wobei die durch die Oberflächentemperatur dieser mindestens einen Lichtquelle (5) verursachte Abwärme von der Steuerungseinrichtung durch die Steuerung des Volumenstroms eines der aktiven Kühlung der mindestens einen Lichtquelle (5) dienenden flüssigen Kühlmediums gesteuert wird.

12. Einrichtung nach Anspruch 8 oder 11, **dadurch gekennzeichnet, dass** in dem wenigstens einen Kultivierungsmodul (1) als Lichtquellen (5) ein oder mehrere Lichtbänder aus LEDs an den Seitenwänden oder an den Seitenwänden und der Decke ihres Gasteils (2) angeordnet sind.

13. Einrichtung nach Anspruch einem der Ansprüche 8 bis 12, **dadurch gekennzeichnet, dass** es sich bei den in dem Gasteil (2) des wenigstens einen Kultivierungsmoduls (1) angeordneten Strukturelementen (6) zur Verzögerung der Abwärtsbewegung der durch Versprühen der Suspension gebildeten Tröpfchen um horizontal angeordnete, strafgespannte flächige Elemente aus einem textilen Vlies oder aus einem textilen feinmaschigen Netz handelt.

14. Einrichtung nach Anspruch einem der Ansprüche 8 bis 13, **dadurch gekennzeichnet, dass** die Einrichtung eine Mehrzahl von jeweils einen Gasteil (2) und einen Flüssigkeitsteil mit einem Flüssigkeitsvorrat (3) aufweisenden Kultivierungsmodulen (1) aufweist, wobei jeweils mehreren oder allen Kultivierungsmodulen (1) eine Zentrifuge (7) zum Ausschleudern für die Ernte von Mikroalgen ausgeschleuster Volumenteile der Suspension gemeinsam zugeordnet ist.

15. Einrichtung nach Anspruch 14, **dadurch gekennzeichnet, dass** diese bis zu 40 Kultivierungsmodule (1) mit jeweils einer Grundfläche von mindestens 250 m² aufweist.

## Claims

1. A process for producing microalgae biomass, in which microalgae contained in a suspension of water and microalgae are phototrophically or mixotrophically cultivated in a continuous circulation in a cultivation module (1) through which the suspension passes multiple times and which comprises a gas portion (2) and a liquid portion having a liquid reservoir (3) with supply of light from at least one artificial light source (5), which light evenly floods the entire gas portion (2) of the cultivation module (1), and of nutrients, wherein volume fractions of the suspension are repeatedly discharged from the cultivation module (1) for harvesting of microalgae and centrifuged by means of a centrifuge (7) and the suspension remaining after centrifugation is supplied back to the cultivation module (1), **characterized in that** volume fractions of the suspension are discharged for harvesting of microalgae whenever the turbidity of the suspension, as established by means of optical sensors (8), exceeds a minimum value, and **in that** the microalgae are cultivated in a climatic chamber which forms the cultivation module (1) and which is operated in a water-economical manner by not only regulating the temperature of the suspension, but also regulating the pH thereof by means of controlled addition of buffer ions and regulating the redox potential of the suspension and hence the microbial contamination thereof by means of control of the supply of light and nutrients and of metered addition of oxygen, and by irradiating the suspension remaining after centrifuging out the microalgae, before it is recycled into the cultivation module (1), with UV light to kill undesirable microbial contamination until a minimum redox potential measured in the suspension is reached.

2. The process as claimed in claim 1, **characterized by** the process steps which are passed through multiple times:
a.) spraying volume fractions of the suspension, which volume fractions contain microalgae to be cultivated and are taken from the liquid reservoir (3) of the liquid portion, in the upper region of the gas portion (2) of the cultivation module (1) into which nutrients, including at least CO₂ as gaseous nutrient, are introduced,
b.) retarding the gravitational downward movement of droplets of the suspension that arise upon spraying and contain the microalgae, by means of suitable structural elements (6) arranged for this purpose in the gas portion (2) of the cultivation module (1),
c.) exposing the microalgae contained in the droplets retarded in their downward movement to the light from LED light sources (5) arranged in the gas portion (2) of the cultivation module (1), which light is tailored to the species of the microalgae to be cultivated with respect to the wavelength thereof and the intensity thereof, and to the introduced CO₂,
d.) recycling downwardly moving, microalgae-containing droplets into the liquid reservoir (3) formed at the bottom of or in a container below the gas portion (2) of the cultivation module (1) after passage through the gas portion (2) of the cultivation module (1),
e.) discharging volume fractions of suspension recycled for respraying in the gas portion (2) of the cultivation module (1) from the liquid reservoir (3) and harvesting microalgae contained in the discharged volume fractions by centrifugation in a centrifuge (7) if the turbidity of the volume fractions of the suspension that are supplied back to the gas portion (2) of the cultivation module (1) for respraying as per process step a), which turbidity is established by means of optical sensors (8), exceeds a minimum value, and also recycling the suspension remaining after centrifugation of the discharged volume fractions into the cultivation module (1) after irradiation with UV light,
wherein
- the pH of the suspension in the liquid portion is measured and is regulated by controlled metered addition of buffer ions and is thereby kept within a pH range between 7 and 8, preferably between 7 and 7.5, that promotes the growth of the microalgae,
- the microbial contamination of the suspension is regulated through monitoring of the redox potential thereof by increasing the supply of nutrients and the light intensity of the LED light sources if the redox potential exceeds an upper limit and reducing the supply of nutrients and the light intensity of the LED light sources (5) and also increasing the metered addition of oxygen that takes place in the liquid portion of the cultivation module (1) if the redox potential falls below a lower limit, wherein the light intensity of the LED light sources (5) is additionally controlled depending on the turbidity of the suspension, which turbidity is determined as per process step e), such that it is adjusted proportionally to the turbidity.

3. The process as claimed in claim 2, **characterized in that** the surface temperature of the LED light sources (5) arranged in the gas portion of the cultivation module is used for temperature control of the suspension, which surface temperature is regulated by reducing or increasing the volumetric flow rate of a cooling medium used for cooling of the LED light sources (5).

4. The process as claimed in any of claims 1 to 3, **characterized in that** calcium ions and/or magnesium ions are metered in as buffer ions for the carbonic acid equilibrium for regulation of the pH of the suspension in the liquid portion of the cultivation module (1).

5. The process as claimed in any of claims 1 to 4, **characterized in that** nitrogen, phosphorus and carbon are supplied as nutrients to the suspension in the liquid portion of the cultivation module (1).

6. The process as claimed in any of claims 1 to 5, **characterized in that** secondary ingredients are formed or enriched in the microalgae by supplying in a controlled quantity to the suspension in the liquid portion of the cultivation module (1) substances or groups of substances that belong to at least one of the following categories:
- nutrients and microbiological contamination for formation of vitamins,
- nutrients for formation of omega-3 fatty acids,
- nutrients for formation of bioactive proteins/peptides,
- minerals to be bound by the microalgae,
- zinc or iron to be bound by the microalgae.

7. The process as claimed in any of claims 1 to 6, **characterized in that** volume fractions of the suspension in the order of magnitude of 15% to 50% of the volume of suspension present in the liquid portion of the cultivation module (1) are discharged from the cultivation module (1) if the minimum value defined for the turbidity of the suspension is exceeded.

8. An installation for production of microalgae biomass, wherein the installation comprises at least
- at least one cultivation module (1) for phototrophic or mixotrophic cultivation of microalgae contained in a suspension with water, consisting of a gas portion (2) and a liquid portion having a liquid reservoir (3) and having at least one nozzle (4) arranged in an upper region of the gas portion as application element for suspension, having at least one light source (5) for emission of artificial light tailored to the species of the microalgae to be cultivated with respect to the wavelength thereof and the intensity thereof, which light source (5) is arranged in the gas portion (2), and also having structural elements (6) arranged in the gas portion (2) for retardation of the gravitational downward movement of droplets that arise upon spraying the suspension,
- inlets and application elements for introduction of CO₂, other nutrients and oxygen into the at least one cultivation module (1),
- at least one centrifuge (7) for centrifugation of microalgae out of volume fractions of the suspension that are discharged from the at least one cultivation module (1) and supplied to the centrifuge (7) for the purpose of harvesting of the microalgae,
- a piping system having pumps (9, 9', 9") for movement of the suspension between the aforementioned components and for supply of substances to be introduced, including nutrients and volume fractions of the suspension, into the cultivation module (1) via the respective application elements,
- a control device for control of the at least one centrifuge (7) and of the aforementioned pumps (9, 9', 9") and application elements in accordance with the results of the evaluation of different sensor signals received from sensors (8) which are arranged in the components of the installation and are operatively connected to the controller,
**characterized in that**
the at least one cultivation module (1) is designed as a climatic chamber which is operated in a water-economical manner by not only regulating the temperature of the suspension by means of the control device on the basis of sensor signals of the sensors (8) arranged in the at least one cultivation module (1), but also regulating the pH of the suspension by means of controlled addition of buffer ions into the liquid portion of the at least one cultivation module (1) and regulating the redox potential of the suspension, which redox potential is measured repeatedly by means of sensors (8), by means of control device-controlled supply of the CO₂, the other nutrients and oxygen into the at least one cultivation module (1) and a control of the light intensity in the gas portion (2) of the at least one cultivation module (1), and by irradiating suspension which remains upon centrifugation of the microalgae out of discharged volume fractions and which is resupplied to the at least one cultivation module (1), before it is reintroduced into the at least one cultivation module (1), with UV light in a controlled manner by means of the control device to kill undesirable microbial contamination until a minimum redox potential measured by sensor in the suspension to be recycled is reached.

9. The installation as claimed in claim 8, **characterized in that** the gas portion (2) of the at least one cultivation module (1) has reflective walls which are mirrored or high-gloss reflective on their inner surface.

10. The installation as claimed in claim 9, **characterized in that** the side walls and the ceiling of the gas portion (2) of the at least one cultivation module (1) are fully mirrored or fully high-gloss reflective on their inner surface.

11. The installation as claimed in claim 8 or 9, **characterized in that** the at least one light source (5) arranged in the gas portion (2) of the at least one cultivation module (1) simultaneously acts as heating for temperature control of the suspension in said at least one cultivation module (1), wherein the waste heat caused by the surface temperature of said at least one light source (5) is controlled by the control device by means of control of the volumetric flow rate of a liquid cooling medium used for active cooling of the at least one light source (5).

12. The installation as claimed in claim 8 or 11, **characterized in that** the at least one cultivation module (1) has arranged on the side walls or on the side walls and the ceiling of the gas portion (2) thereof one or more light strips composed of LEDs as light sources (5).

13. The installation as claimed in any of claims 8 to 12, **characterized in that** the structural elements (6) for retardation of the downward movement of the droplets formed by spraying of the suspension, which structural elements (6) are arranged in the gas portion (2) of the at least one cultivation module (1), are horizontally arranged, tautly stretched planar elements composed of a textile nonwoven or of a textile fine-meshed mesh.

14. The installation as claimed in any of claims 8 to 13, **characterized in that** the installation comprises a plurality of cultivation modules (1) which each comprise a gas portion (2) and a liquid portion having a liquid reservoir (3), wherein a centrifuge (7) for centrifugation of volume fractions of the suspension discharged for harvesting of microalgae is jointly assigned to multiple or all cultivation modules (1).

15. The installation as claimed in claim 14, **characterized in that** it comprises up to 40 cultivation modules (1) having a base area of at least 250 m² in each case.

## Revendications

1. Procédé pour la production de biomasse à base de microalgues, dans lequel des microalgues contenues dans une suspension d'eau et de microalgues sont cultivées en circulation continue en mode phototrophe ou mixotrophe dans un module de culture (1) traversé plusieurs fois par la suspension, comportant une partie gazeuse (2) et une partie liquide avec une réserve de liquide (3), avec un apport de lumière d'au moins une source de lumière artificielle (5) irradiant de façon uniforme toute la partie gazeuse (2) du module de culture (1) et de nutriments, dans lequel des fractions de volume de la suspension sont dérivées de façon répétée hors du module de culture (1) pour la récolte de microalgues et centrifugés au moyen d'une centrifugeuse (7) et la suspension restant après la centrifugation est ramenée au module de culture (1), **caractérisé en ce que** la dérivation de fractions de volume de la suspension pour la récolte de microalgues est effectuée si la turbidité de la suspension constatée au moyen de capteurs optiques (8) dépasse un minimum et **en ce que** les microalgues sont cultivées dans une chambre climatique formant le module de culture (1) qui est pilotée, du point de vue de la gestion de l'eau, avec, outre une régulation de la température de la suspension, une régulation de son pH par l'ajout contrôlé d'ions tampons et une régulation du potentiel d'oxydoréduction de la suspension et ainsi de la contamination microbienne par un contrôle des apports de lumière et de nutriments et un ajout d'oxygène, et avec une irradiation avec une lumière UV de la solution restant après la centrifugation des microalgues avant son renvoi dans l'au moins un module de culture (1) afin d'éliminer des contaminations microbiennes indésirables, jusqu'à ce qu'un potentiel d'oxydoréduction mesuré dans la suspension atteigne un minimum.

2. Procédé selon la revendication 1, **caractérisé en ce qu'**il comporte les étapes de procédé suivantes, qui sont exécutées plusieurs fois :
a) pulvérisation de fractions de volume de la suspension contenant des microalgues à cultiver prélevées dans la réserve de liquide (3) de la partie liquide dans la partie supérieure de la partie gazeuse (2) du module de culture (1), dans laquelle sont amenés des nutriments, dont au moins du CO₂ constituant un nutriment gazeux ;
b) ralentissement du mouvement descendant par gravité lors de la pulvérisation de gouttelettes de la suspension contenant des microalgues au moyen d'éléments structurels (6) appropriés disposés pour cela dans la partie gazeuse (2) du module de culture (1) ;
c) exposition des microalgues contenues dans les gouttelettes ralenties dans leur mouvement à de la lumière de sources lumineuses à DEL (5) disposées dans la partie gazeuse (2) du module de culture (1), dont la longueur d'onde et l'intensité sont adaptées à l'espèce des microalgues à cultiver, et au CO₂ introduit ;
d) renvoi des gouttelettes en mouvement descendant contenant les microalgues dans la réserve de liquide (3) formée dans le fond ou dans un contenant situé en dessous de la partie gazeuse (2) du module de culture (1) après le passage dans la partie gazeuse (2) du module de culture (1) ;
e) dérivation de fractions de volume hors de la réserve de liquide (3) en vue d'une nouvelle pulvérisation de suspension ramenée dans la partie gazeuse (2) du module de culture (1) et récolte des microalgues contenues dans les fractions de volume dérivées par centrifugation dans une centrifugeuse (7) si la turbidité constatée au moyen de capteurs optiques (8) des fractions de volume de la suspension ramenées à la partie gazeuse (2) du module de culture (1) en vue d'une nouvelle pulvérisation selon l'étape de procédé a) dépasse un minimum, et renvoi de la suspension restant après la centrifugation des fractions de volume dérivées dans le module de culture (1) après une irradiation avec de la lumière UV,
- le pH de la suspension dans la partie liquide étant mesuré et régulé par un ajout contrôlé d'ions tampons et ainsi maintenu dans une plage de pH de 7 à 8, de préférence de 7 à 7,5, favorable à la croissance des microalgues,
- la contamination microbienne de la suspension étant régulée par une surveillance de son potentiel d'oxydoréduction en augmentant l'apport de nutriments et l'intensité lumineuse des sources lumineuses à DEL si une limite supérieure du potentiel d'oxydoréduction est dépassée et en réduisant l'apport de nutriments et l'intensité lumineuse des sources lumineuses à DEL (5) si une limite inférieure du potentiel d'oxydoréduction n'est pas atteinte, et en augmentant l'ajout d'oxygène dans la partie liquide du module de culture (1), l'intensité lumineuse des sources lumineuses à DEL (5) étant en outre contrôlée en fonction de la turbidité de la suspension déterminée selon l'étape e) du procédé de façon à l'ajuster de façon proportionnelle à la turbidité.

3. Procédé selon la revendication 2, **caractérisé en ce qu'**il utilise pour le maintien à température de la suspension la température de surface des sources lumineuses à DEL (5) disposées dans la partie gazeuse du module de culture, qui est régulée par une réduction ou une augmentation du débit volumique d'un fluide de refroidissement utilisé pour refroidir les sources lumineuses à DEL (5).

4. Procédé selon l'une des revendications 1 à 3, **caractérisé en ce que** des ions de calcium et/ou de magnésium sont ajoutés pour servir d'ions tampons pour l'équilibre du gaz carbonique, afin de réguler le pH de la suspension dans la partie liquide du module de culture (1).

5. Procédé selon l'une des revendications 1 à 4, **caractérisé en ce que** de l'azote, du phosphore et du carbone sont apportés comme nutriments à la suspension dans la partie liquide du module de culture (1).

6. Procédé selon l'une des revendications 1 à 5, **caractérisé en ce que** des ingrédients secondaires sont formés ou ajoutés dans les microalgues par l'ajout à la suspension, dans la partie liquide du module de culture (1), d'une quantité contrôlée de substances ou de groupes de substances qui appartiennent à au moins une des catégories suivantes :
- nutriments et contaminants microbiologiques pour la synthèse de vitamines,
- nutriments pour la synthèse d'acides gras oméga-3,
- nutriments pour la synthèse de protéines/peptides bioactifs,
- minéraux à fixer par les microalgues,
- zinc ou fer à fixer par les microalgues.

7. Procédé selon l'une des revendications 1 à 6, **caractérisé en ce que**, quand la turbidité de la suspension dépasse le minimum fixé, des fractions de volume de la suspension sont dérivées du module de culture (1) dans un ordre de grandeur de 15 % à 50 % du volume de suspension qui se trouve dans la partie liquide du module de culture (1).

8. Installation pour la production de biomasse à base de microalgues, laquelle installation comporte au moins :
- au moins un module de culture (1) pour la culture phototrophe ou mixotrophe de microalgues contenues dans une suspension avec de l'eau, composé d'une partie gazeuse (2) et d'une partie liquide avec une réserve de liquide (3) et avec au moins un gicleur (4) disposé dans la région supérieure de la partie gazeuse pour servir d'organe de déversement de la suspension, avec au moins une source lumineuse (5) disposée dans la partie gazeuse (2) pour émettre une lumière artificielle dont la longueur d'onde et l'intensité sont adaptées à l'espèce des microalgues à cultiver et avec des éléments structurels (6) disposés dans la partie gazeuse (2) afin de ralentir le mouvement descendant par gravité de gouttelettes formées lors de la pulvérisation de la suspension ;
- des entrées et des organes de déversement pour l'introduction de CO₂, d'autres nutriments et d'oxygène dans l'au moins un module de culture (1) ;
- au moins une centrifugeuse (7) pour centrifuger des microalgues à partir de fractions de volume de la suspension qui sont dérivées hors de l'au moins un module de culture (1) et amenées à la centrifugeuse (7) en vue de la récolte des microalgues ;
- un système de tuyauteries avec des pompes (9, 9', 9") pour déplacer la suspension entre les composants précédemment cités et l'introduire dans le module de culture (1) via les organes de déversement correspondants des substances à introduire, y compris des nutriments et des fractions de volume de la suspension ;
- une installation de commande pour la commande de l'au moins une centrifugeuse (7) et desdites pompes (9, 9', 9") et organes de déversement en fonction des résultats de l'analyse de différents signaux de capteur reçus de capteurs (8) disposés dans les composants de l'installation et en liaison active avec la commande,
**caractérisée en ce que**
l'au moins un module de culture (1) est conformé comme une chambre climatique qui est pilotée, du point de vue de la gestion de l'eau, avec, outre une régulation de la température de la suspension au moyen de l'installation de commande sur la base de signaux de capteur des capteurs (8) disposés dans l'au moins un module de culture (1), une régulation du pH de la suspension par l'ajout contrôlé d'ions tampons dans la partie liquide de l'au moins un module de culture (1) et une régulation du potentiel d'oxydoréduction de la suspension mesuré de façon répétée au moyen de capteurs (8) par un apport régulé par l'installation de commande de CO₂, des autres nutriments et d'oxygène dans l'au moins un module de culture (1) ainsi que de l'intensité lumineuse dans la partie gazeuse (2) de l'au moins un module de culture (1), et avec une irradiation avec une lumière UV de solution restant lors de la centrifugation des microalgues et renvoyée dans l'au moins un module de culture (1) avant sa réintroduction dans l'au moins un module de culture (1) afin d'éliminer des contaminations microbiennes indésirables, sous le contrôle de l'installation de commande, jusqu'à ce qu'un potentiel d'oxydoréduction mesuré par les capteurs dans la suspension à renvoyer atteigne un minimum.

9. Installation selon la revendication 8, **caractérisée en ce que** la partie gazeuse (2) de l'au moins un module de culture (1) comporte des parois réfléchissantes dont la paroi intérieure est dotée d'une couche réfléchissante ou d'un brillant spéculaire.

10. Installation selon la revendication 9, **caractérisée en ce que** les parois latérales et la paroi supérieure de la partie gazeuse (2) de l'au moins un module de culture (1) sont entièrement tapissées de miroirs ou entièrement brillantes et réfléchissantes sur leur face intérieure.

11. Installation selon la revendication 8 ou 9, **caractérisée en ce que** l'au moins une source lumineuse (5) disposée dans la partie gazeuse (2) de l'au moins un module de culture (1) sert en même temps de chauffage pour le maintien à température de la suspension dans cet au moins un module de culture (1), la chaleur dissipée produite par la température de surface de cette au moins une source lumineuse (5) étant contrôlée par l'installation de commande pour contrôler le débit volumique d'un liquide de refroidissement servant au refroidissement actif de l'au moins une source lumineuse (5).

12. Installation selon la revendication 8 ou 11, **caractérisée en ce qu'**un ou plusieurs bandeaux lumineux composés de DEL sont disposés dans l'au moins un module de culture (1), sur les parois latérales ou sur les parois latérales et la partie supérieure de sa partie gazeuse (2), pour servir de sources lumineuses (5).

13. Installation selon l'une des revendications 8 à 12, **caractérisée en ce que** les éléments structurels (6) disposés dans la partie gazeuse (2) de l'au moins un module de culture (1) pour ralentir le mouvement descendant des gouttelettes formées par la pulvérisation de la suspension sont des éléments plans tendus, disposés horizontalement, faits d'un non-tissé textile ou d'un filet textile à mailles fines.

14. Installation selon l'une des revendications 8 à 13, **caractérisée en ce qu'**elle comporte plusieurs modules de culture (1) comportant chacun une partie gazeuse (2) et une partie liquide avec une réserve de liquide (3), plusieurs ou tous les modules de culture (1) étant associés respectivement à une centrifugeuse (7) commune destinée à centrifuger des fractions de volume de la suspension dérivées en vue de la récolte de microalgues.

15. Installation selon la revendication 14, **caractérisée en ce que** qu'elle comporte jusqu'à 40 modules de culture (1) ayant chacun une aire d'au moins 250 m².
